# EUROPEAN PATENT APPLICATION

(11) **EP 2 618 021 A2**
(43) Date of publication of application: **24.07.2013**
(21) Application number: 13151969.6
(22) Date of filing: 18.01.2013
(51) Int. Cl.: F16F 15/027, A61B 6/03, F16C 32/06

(54) **Vibration control apparatuses, vibration control methods, and computed tomography scanners**

(30) Priority: 20.01.2012 KR 20120006524
(71) Applicant: Samsung Electronics Co., Ltd, Gyeonggi-do 443-742 (KR)
(72) Inventor: Lee, Ja Woo, Gyeonggi-do (KR)
(74) Representative: Mounteney, Simon James

(57) **Abstract**

A vibration control apparatus may include a fluid bearing (402) provided around a rotating body (102) to form a fluid bearing gap (504) between the fluid bearing (402) and the rotating body (102); and a pressure regulator (604) configured to variably control a pressure of the fluid bearing (402), based on imbalance information of the rotating body (102), to compensate for imbalance of the rotating body (102). A computed tomography scanner may include a gantry (102) configured to generate a computed tomography image while rotating around a test subject; a fluid bearing (402) provided around the gantry (102) to form a fluid bearing gap (504) between the fluid bearing (402) and the gantry (102); and/or a pressure regulator (604) configured to variably control a pressure of the fluid bearing (402), based on imbalance information of the gantry (102), to compensate for imbalance of the gantry (102).

## Description

### BACKGROUND

### 1. Field

The present invention relates to vibration controls. Example embodiments may relate to vibration controls configured to control vibration caused by eccentric forces of rotating bodies.

### 2. Description of Related Art

A computed tomography (CT) scanner may include a gantry, a table for a patient, and a console. When a patient passes through a test area at a central portion of the gantry while the patient is laid on the table, radiation may be generated to be projected on the body of the patient while the gantry is rotated. By reconstructing an image that is obtained from the above by use of a computer, a cross-sectional image of an inside the body of the patient may be generated.

Inside the gantry of the CT scanner, a radiation source configured to generate radiation, a radiation detection unit configured to detect the radiation that is emitted from the radiation source and is projected to a patient to generate an electric signal that corresponds to the detected radiation, a high-voltage generating unit configured to supply the energy needed to generate and emit radiation from the radiation source, and various apparatuses needed for a CT scanning may be provided.

The installation position of each of the various apparatuses having the radiation source, the radiation detection unit, and the high-voltage generating unit may be asymmetrical to each other, and the weight of each apparatus may be different from the weight of other apparatuses. Therefore, when the gantry is rotated, because of the asymmetrical structure discussed above, an eccentricity may occur. Thus, a probability of having a vibration at the gantry is large as a result. Since the vibration of the gantry may reduce the quality of the image of the test from the CT scanner, the vibration of the gantry should be reduced or removed. The vibration that occurs when a rotating structure is rotated, not to mention the CT scanner, is closely related to the performance and the durability of the structure and, therefore, it may be important to remove such vibration in every rotating structure.

### SUMMARY

The present invention sets out to provide vibration control apparatuses capable of reducing vibration due to imbalance of a rotating body, when the rotating body is rotated.

Accordingly, the invention provides a vibration control apparatus that comprises a fluid bearing provided around a rotating body to form a fluid bearing gap between the fluid bearing and the rotating body; and/or a pressure regulator configured to variably control a pressure of the fluid bearing, based on imbalance information of the rotating body, to compensate for imbalance of the rotating body.

Thus, by properly regulating pressure of a bearing that supports the rotating body according to the vibration status of the rotating body, the vibration can be controlled.

In some example embodiments, the fluid bearing may be an air bearing.

In some example embodiments, the pressure regulator may be an electro-pneumatic regulator.

In some example embodiments, the vibration control apparatus may comprise a plurality of fluid bearings. A pressure of each of the plurality of fluid bearings may be variably controlled in an independent manner.

In some example embodiments, a vibration control method may comprise rotating a rotating body of a rotating structure; forming a fluid bearing gap between the rotating body and a fluid bearing by providing a fluid bearing around the rotating body; and/or controlling a pressure of the fluid bearing in a variable manner, based on imbalance information of the rotating body, to compensate for imbalance of the rotating body.

In some example embodiments, the fluid bearing may be an air bearing.

In some example embodiments, the pressure of the fluid bearing may be changed by using a pressure regulator.

In some example embodiments, the pressure regulator may be an electro-pneumatic regulator.

In some example embodiments, the fluid bearing may comprise a plurality of fluid bearings. A pressure of each of the plurality of fluid bearings may be variably controlled in an independent manner.

In some example embodiments, a computed tomography scanner may comprise a gantry configured to generate a computed tomography image while rotating around a test subject; a fluid bearing provided around the gantry to form a fluid bearing gap between the fluid bearing and the gantry; and/or a pressure regulator configured to variably control a pressure of the fluid bearing, based on imbalance information of the gantry, to compensate for imbalance of the gantry.

In some example embodiments, an apparatus to compensate for imbalance of a body may comprise the body; a fluid bearing at least partially around the body; and/or a pressure regulator. The apparatus may be configured to create a fluid bearing gap between the fluid bearing and the body. The pressure regulator may be configured to variably control a pressure of the fluid bearing, based on imbalance information of the body, to compensate for imbalance of the body.

In some example embodiments, the fluid bearing may extend completely around the body.

In some example embodiments, the fluid bearing may be an air bearing.

In some example embodiments, the pressure regulator may be an electro-pneumatic regulator.

In some example embodiments, the fluid bearing may be configured to support a weight of the body.

In some example embodiments, the apparatus may comprise a plurality of fluid bearings. A pressure of each of the plurality of fluid bearings may be variably controlled in an independent manner.

In some example embodiments, the fluid bearings may be configured to support a weight of the body.

In some example embodiments, the fluid bearings may be equally spaced around the body.

In some example embodiments, the fluid bearings may act on the body at equal intervals around the body.

In some example embodiments, the apparatus may be configured to create the fluid bearing gap between the fluid bearing and the body before the body rotates.

In some example embodiments, the apparatus may be configured to maintain the fluid bearing gap between the fluid bearing and the body when the body rotates.

In some example embodiments, the fluid bearing may be configured to support a weight of the body when the body rotates.

In some example embodiments, the apparatus may be configured to prevent rotation of the body before the fluid bearing gap is created.

In some example embodiments, the apparatus may be configured to allow rotation of the body after the fluid bearing gap is created.

In some example embodiments, the apparatus may be configured to stop rotation of the body before the fluid bearing gap is lost.

At least some of the above and other features of the invention are set out in the claims.

### BRIEF DESCRIPTION OF THE DRAWINGS

The above and/or other aspects and advantages of the invention will become more apparent and more readily appreciated upon making reference to the following detailed description of example embodiments, in conjunction with the accompanying drawings, in which:
FIG. 1 is a drawing showing a computed tomography (CT) scanner according to an embodiment of the invention;
FIG. 2 is a drawing showing an inside structure of a gantry of the CT scanner of FIG. 1;
FIG. 3 is a drawing showing a control system of a gantry of the CT scanner of FIG. 1;
FIG. 4 is a drawing showing a coupling structure of a fluid bearing of a gantry of the CT scanner of FIG. 1;
FIG. 5 is a cross-sectional view of the air bearing of FIG. 4;
FIG. 6 is a drawing showing a connection status of an electro-pneumatic regulator unit configured to regulate pressure of the air bearing of FIG. 4;
FIG. 7 is a drawing showing the pressure regulation status of the air bearing of FIG. 4 using an electro-pneumatic regulator;
FIG. 8 is a drawing showing a control system of an electro-pneumatic regulator; and
FIG. 9 is a drawing showing a vibration control method of the CT scanner of FIG. 1.

### DETAILED DESCRIPTION

Example embodiments of the invention will now be described more fully with reference to the accompanying drawings. However, the invention may be embodied in many different forms and should not be construed as being limited to the embodiments set forth herein. Rather, these example embodiments are provided so that this disclosure will be thorough. In the drawings, the thicknesses of layers and regions may be exaggerated for clarity.

It will be understood that when an element is referred to as being "on," "connected to," "electrically connected to," or "coupled to" to another component, it may be directly on, connected to, electrically connected to, or coupled to the other component or intervening components may be present. In contrast, when a component is referred to as being "directly on," "directly connected to," "directly electrically connected to," or "directly coupled to" another component, there are no intervening components present. As used herein, the term "and/or" includes any and all combinations of one or more of the associated listed items.

It will be understood that although the terms first, second, third, etc., may be used herein to describe various elements, components, regions, layers, and/or sections, these elements, components, regions, layers, and/or sections should not be limited by these terms. These terms are only used to distinguish one element, component, region, layer, and/or section from another element, component, region, layer, and/or section. For example, a first element, component, region, layer, and/or section could be termed a second element, component, region, layer, and/or section without departing from the teachings of example embodiments.

Spatially relative terms, such as "beneath," "below," "lower," "above," "upper," and the like may be used herein for ease of description to describe the relationship of one component and/or feature to another component and/or feature, or other component(s) and/or feature(s), as illustrated in the drawings. It will be understood that the spatially relative terms are intended to encompass different orientations of the device in use or operation in addition to the orientation depicted in the figures.

The terminology used herein is for the purpose of describing particular example embodiments only and is not intended to be limiting of the invention. As used herein, the singular forms "a," "an," and "the" are intended to include the plural forms as well, unless the context clearly indicates otherwise. It will be further understood that the terms "comprises," "comprising," "includes," and/or "including," when used in this specification, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which example embodiments belong. It will be further understood that terms, such as those defined in commonly used dictionaries, should be interpreted as having a meaning that is consistent with their meaning in the context of the relevant art and should not be interpreted in an idealized or overly formal sense unless expressly so defined herein.

Reference will now be made to example embodiments of the invention, which are illustrated in the accompanying drawings, wherein like reference numerals may refer to like components throughout.

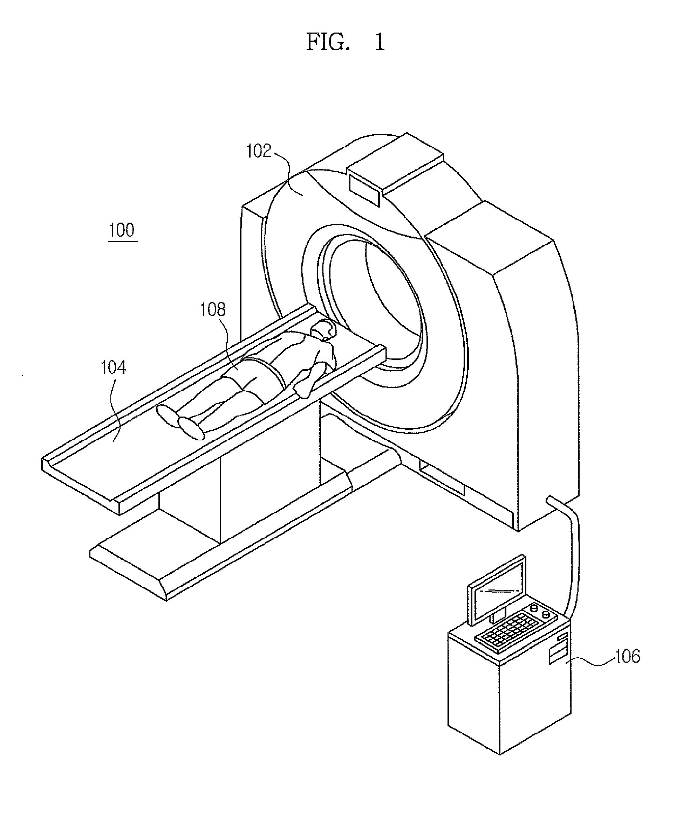

FIG. 1 is a drawing showing a computed tomography (CT) scanner according to an example embodiment of the invention. According to the invention, a rotating body may interact with a vibration control apparatus. In order to reduce the vibration occurred by an imbalance of the rotating body when the rotating body is rotated, vibration may be restricted by controlling the pressure of a fluid bearing that is configured to support the rotating body according to the vibration status of the rotating body. In this embodiment, the fluid bearing is an air bearing, but other types of fluid bearing fall within the scope of the invention. In this embodiment, gantry 102 of CT scanner 100 of FIG. 1 is such a rotating body.

As shown in FIG. 1, CT scanner 100 includes gantry 102, table 104 for patient 108, and console 106. Inside the gantry 102, apparatuses are provided to project radiation and/or to detect the radiation, and gantry 102 is installed so as to rotate together with the apparatuses. When patient 108 laid on the table 104 passes through a test area at a central portion of gantry 102, the gantry 102 may generate radiation and/or project radiation on the body of patient 108, and by reconstructing an image that may be obtained from the above, by use of a computer, a cross-sectional image of an inside the body of patient 108 may be generated. Console 106 is provided for a user to manipulate the CT scanner 100, and includes a manipulation panel and a monitor. Depending on the type thereof, the CT scanner 100 may use ultrasonic waves instead of radiation.

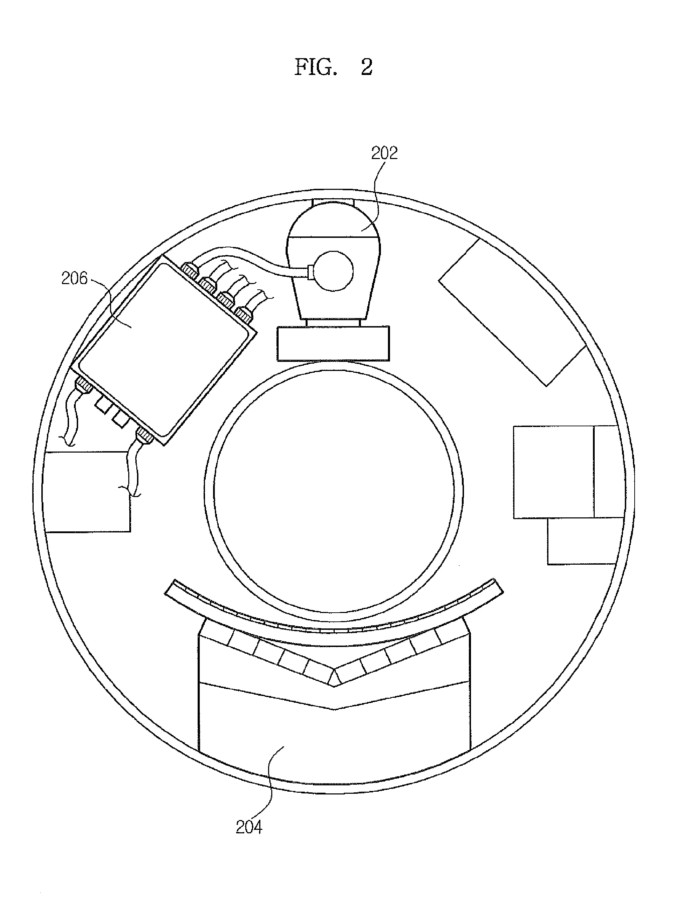

FIG. 2 is a drawing showing an inside structure of gantry 102 of CT scanner 100 of FIG. 1. As shown on FIG. 2, the inside of the gantry 102 is provided with radiation source 202 configured to generate radiation, radiation detection unit 204 configured to detect the radiation emitted from radiation source 202 and projected to patient 108 so that an electrical signal corresponding to the detected radiation is generated, high-voltage generating unit 206 configured to provide the energy needed for radiation source 202 to generate and emit radiation, and/or various apparatuses needed for the CT scanning. As shown in FIG. 2, the installation positions of each of the various apparatuses including radiation source 202, radiation detection unit 204, and high-voltage generating unit 206 are asymmetrical with respect to each other. Furthermore, the respective weight of the each apparatus may be different from each other. When the weights of radiation source 202, radiation detection unit 204, and high-voltage generating unit 206 are different to each other, in the asymmetrical disposition structure shown in FIG. 2, the possibility of having a vibration at gantry 102 caused by the eccentricity, when gantry 102 is rotated, is large. In this embodiment, control of the vibration of the rotating body serves to reduce or prevent the vibration caused by such eccentricity when the gantry 102 is rotated.

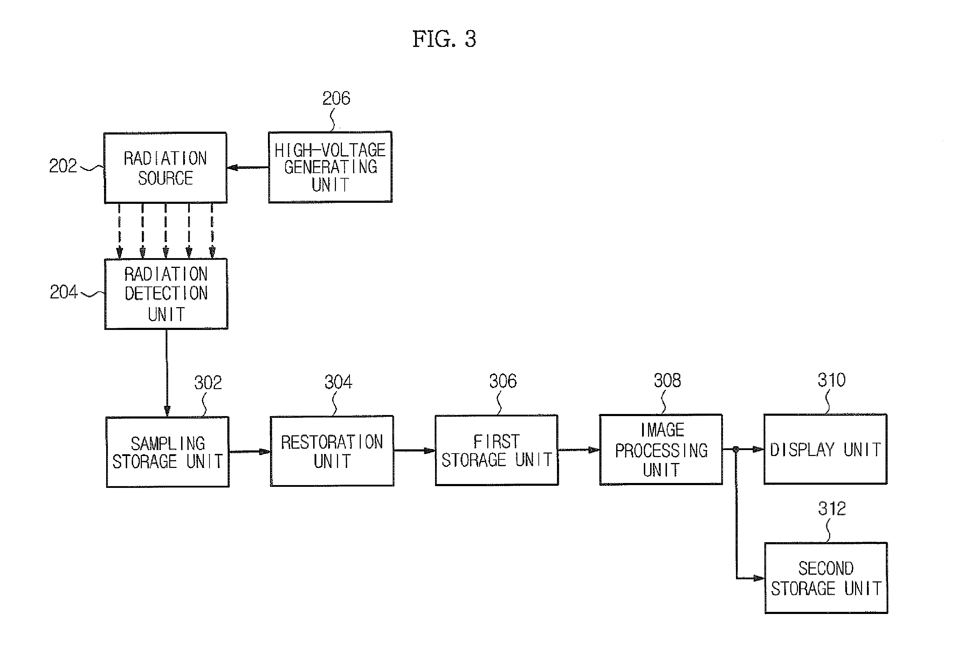

FIG. 3 is a drawing showing a control system of gantry 102 of CT scanner 100 of FIG. 1. As shown in FIG. 3, radiation detection unit 204 may detect the radiation emitted from radiation source 202 and/or projected to patient 108, and may generate an electrical signal corresponding to the detected radiation. The electrical signal generated at radiation detection unit 204 may be stored as projection data at sampling storage unit 302 through a sampling process and, then, image information may be made as restoration unit 304 reconstructs (restores) the projection data of sampling storage unit 302. The image information made by restoration unit 304 may be stored at first image storage unit 306. Image processing unit 308, by processing the image information stored at first image storage unit 306, may produce various forms of image such as a slice, a projection, and/or a three-diznensional (3D) rendering. The image produced by image processing unit 308 may be displayed through display unit 310 and/or may be stored at second image storage unit 312.

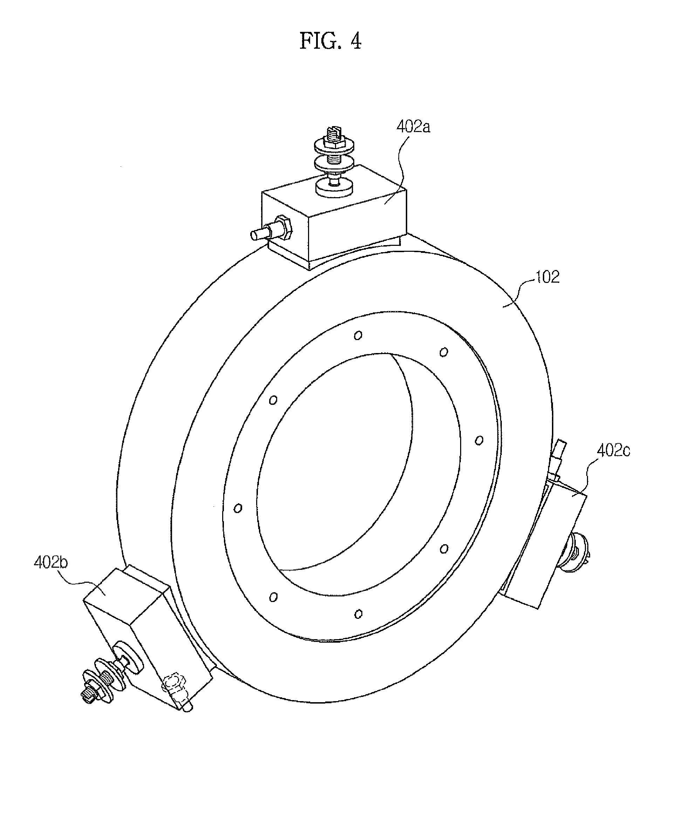

FIG. 4 is a drawing showing a coupling structure of a fluid bearing of gantry 102 of FIG. 1. As shown in FIG. 4, when CT scanner 100 rotates around patient 108 to scan the body of patient 108, air bearings 402a, 402b, and 402c may be used to reduce or eliminate the noise and/or friction generated from the rotation of CT scanner 100. When air bearings 402a, 402b, and 402c are being respectively mentioned in some example embodiments, the reference numerals are used separately, such as 402a, 402b, and 402c, and when air bearings 402a, 402b, and 402c are being mentioned as a whole, reference numeral 402 will be representatively used. Air bearings 402 are provided as an example embodiment of fluid bearings, and any other bearings using other fluid substances other than air may be used. Air bearings 402 may be configured to eject air at a high pressure and, by the force of the ejected air, the rotating body (gantry 102) may be rotated while supported in between air bearings 402.

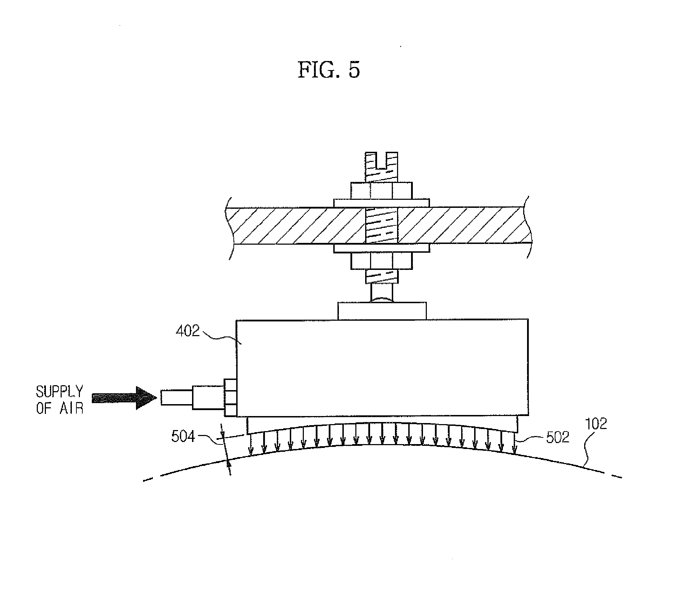

FIG. 5 is an air bearing 402 of FIG. 4. As shown on FIG. 5, air bearings 402, by using a thin, compressed air film 502 (a fluid film), may form a weight-bearing contact surface with little or no friction thereof with respect to a contact surface of gantry 102. Air bearings 402 do not therefore make direct contact with the contact surface of gantry 102 and, thus, air bearings 402 may be able to reduce or avoid friction, wear, and/or lubrication, all of which may occur at different types of bearings, and may be provided with significant merits in the fields of precision position control and high-velocity application. A fluid bearing gap 504 is formed in between air bearings 402 and gantry 102. In some embodiments, air bearings 402 are used and, thus, the fluid bearing gap 504 becomes the air bearing gap. No contact and little or no friction occurs while gantry 102 is rotatively supported by air bearings 402 as a consequence of the fluid bearing gap 504, in between air bearings 402 and gantry 102. The compressed air film 502 may be made by the air that is discharged through an orifice or a porous media that is formed at air bearings 402 so as to limit the flow of the air. In some embodiments, the discharging pressure of the air being discharged from air bearings 402 is variably controlled in order to control the vibration that occurs while the rotating body (gantry 102) is rotated.

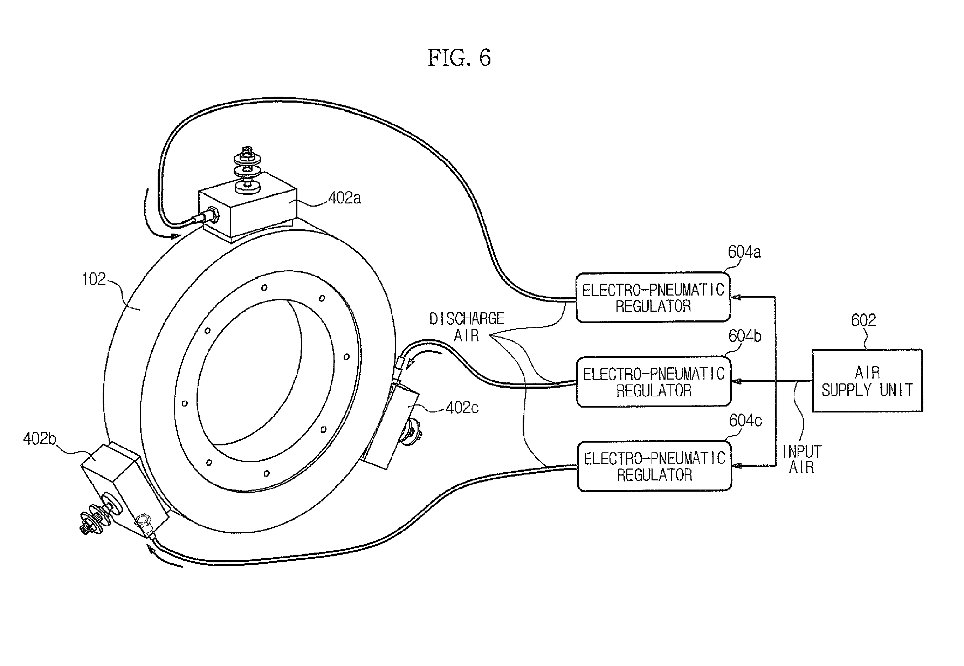

FIG. 6 is a drawing showing a connection status of electro-pneumatic regulator units 604a, 604b, and 604c configured to control the pressure of the air bearing. When electro-pneumatic regulator units 604a, 604b, and 604c are being respectively mentioned in some example embodiments, the reference numerals are used separately, such as 604a, 604b, and 604c, and when electro-pneumatic regulator units 604a, 604b, and 604c are being mentioned as a whole, reference numeral 604 will be representatively used. Electro-pneumatic regulator units 604 may each be pressure regulators configured to control the pressure being discharged from air bearings 402, and also may be configured to generate the pressure that is in proportion to the magnitude of an electrical signal. The air, supplied from air supply unit 602, may be introduced to each of air bearings 402 through a respective pipe. In the course of the air being delivered to each of air bearings 402 from air supply unit 602, electro-pneumatic regulator units 604 may allow the pressure of the air being discharged from each of air bearings 402 to be controlled. Electro-pneumatic regulator units 604 may be installed so that a variable control of the pressure being discharged in an independent manner may be achieved with respect to each of air bearings 402. Electro-pneumatic regulator units 604 may variably control the pressure of the air being discharged by comparing a pressure value (that may or may not be predetermined) with an output pressure, so that the output pressure follows the pressure value (that may or may not be predetermined). As for the pressure value (that may or may not be predetermined) as example, the such may be referred to as a target pressure control value of each of air bearings 402 that may be needed to reduce the vibration of the rotating body (gantry 102).

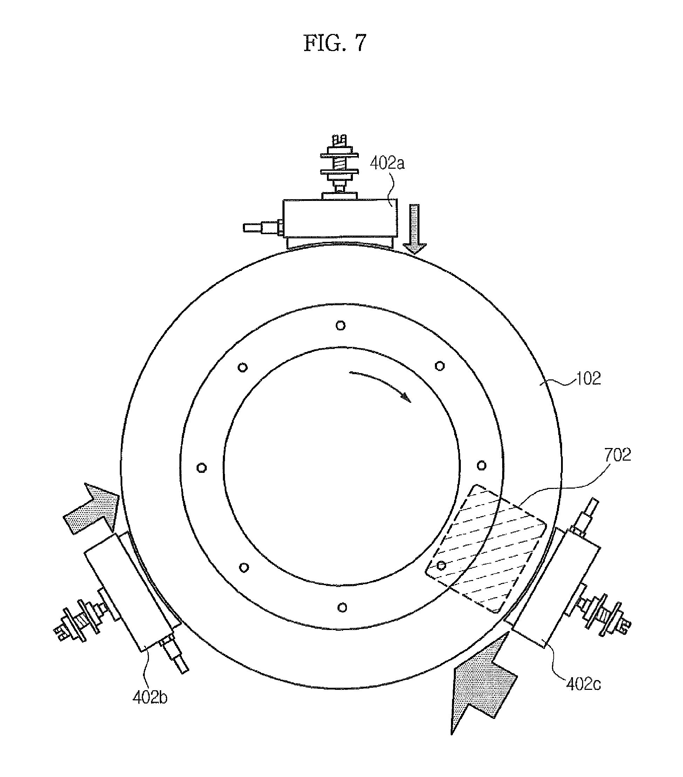

FIG. 7 is a drawing showing the pressure control status of an air bearing using an electro-pneumatic regulator. In FIG. 7, the illustration shown with the reference numeral '702' refers to the state in which the mass center of gantry 102 is deviated toward one side, that is, eccentrically positioned toward one side. In some example embodiments, by increasing the discharging pressure of the air (shown with an arrow) of air bearing 402c, which is closer to the portion at which the eccentricity as such is occurred, the impact of the eccentricity, such as vibration and noise, as well as friction, may be minimized. In addition, by increasing the discharging pressure of the air of air bearing 402b to a certain degree, in cooperation with air bearing 402c, a larger weight formed at a lower portion of gantry 102 may be accommodated. As the above, by variably controlling the discharging pressure of the air of each of air bearings 402 in an independent manner, an optimal pressure control may be realized while considering the vibration control of gantry 102 that is configured to rotate. The degree of the eccentricity and/or vibration of the rotating body, that is, gantry 102 may be checked in advance by conducting a test rotation of gantry 102 during a development stage of the CT scanner 100. By setting the degree of the pressure of each of air bearings 402, which may be configured to restrict the vibration by offsetting the eccentricity, the relationship of the degree of the pressure at each of air bearings 402 with respect to the rotating position (the angle) and/or the speed of gantry 102 may be put into a data as imbalance information, and by referring to the imbalance information as such, a control unit of CT scanner 100, while an actual operation of CT scanner 100 is performed, may variably control the pressure of each of air bearings 402. The rotating position (the angle) of gantry 102 may use an encoder of a motor that drives gantry 102.

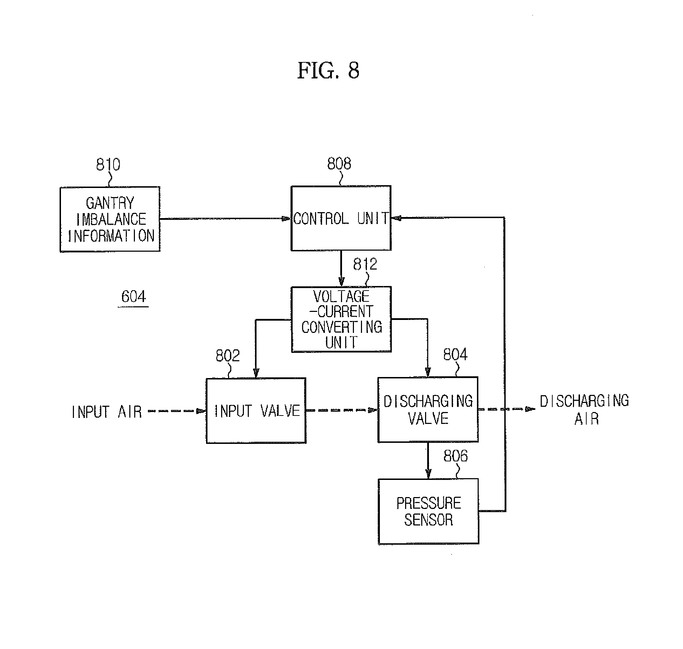

FIG. 8 is a drawing showing a control system of an electro-pneumatic regulator. As shown in FIG. 8, the air being introduced to air bearings 402 may be discharged through input valve 802 and discharging valve 804. The pressure of the air being discharged through discharging valve 804 may be detected by pressure sensor 806, and/or the detected value may be provided to control unit 808. Control unit 808, through imbalance information 810 of gantry 102, may secure the target pressure of each of air bearings 402 needed to stabilize the rotation of gantry 102. Control unit 808, by comparing the target pressure with the detected pressure of pressure sensor 806, may calculate the difference of the target pressure and the detected pressure and, on the basis of the difference that is calculated, may control input valve 802 and/or discharging valve 804 to variably control the discharging pressure of the air, so that the discharging pressure of the air of discharging valve 804 may follow the target pressure. Voltage-current converting unit 812, by converting the pressure control signal being generated at control unit 808 into a current signal having a magnitude corresponding to a voltage level of the pressure control signal and/or by providing the converted current signal to input valve 802 and discharging valve 804, may enable a pressure control to take place at each of input valve 802 and/or discharging valve 804.

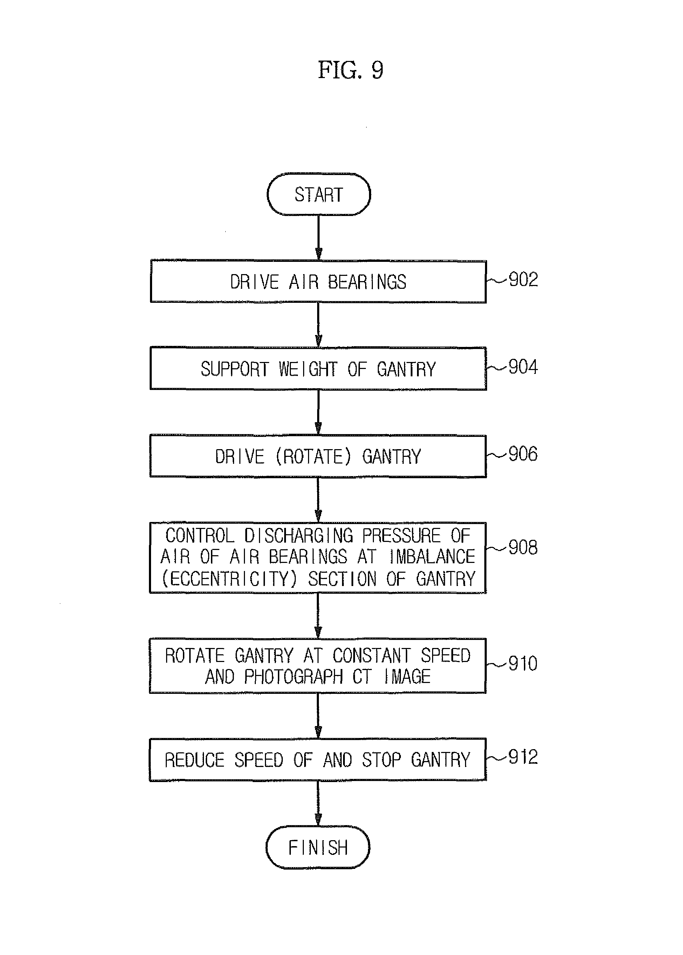

FIG. 9 is a drawing showing a vibration control method of CT scanner 100 of FIG. 1. As shown on FIG. 9, while the operation of gantry 102 is stopped, prior to patient 108 entering a test area at a central portion of gantry 102 while patient 108 is laid on table 104, air bearings 402 may be operated to prepare for the rotation of gantry 102 (902). By operating air bearings 402, the air bearing gap may be formed in between air bearings 402 and gantry 102, so that air bearings 402 may be able to support the weight of gantry 102 (904). In a state of air bearings 402 supporting the weight of gantry 102, gantry 102 may be operated and/or rotated for the CT scanning (906). While gantry 102 is rotated, table 104 passes through the test area at a central portion of gantry 102.

While gantry 102 is being rotated, at the section where an imbalance (eccentricity) of gantry 102 is present, the discharging pressure of the air of air bearings 402 may be controlled to reduce the imbalance of gantry 102 (908). The controlling of the discharging pressure of the air of air bearings 402 may be performed by increasing the discharging pressure of the air of air bearings 402 at the position where the degree of the eccentricity of gantry 102 is large, and/or by decreasing or maintaining the discharging pressure of the air of air bearings 402 at the position where the degree of the eccentricity of gantry 102 is relatively small, so that the bearing gap in between rotating gantry 102 and all air bearings 402 may be formed at a constant rate at all times. For the above, the discharging pressure of the air from each of the three units of air bearings 402a, 402b, and 402c may be variably controlled in an independent manner. Through the variable control of the discharging pressure of the air of air bearings 402, the vibration of rotating gantry 102 may be controlled.

While controlling the discharging pressure of the air of air bearings 402 configured to restrict the imbalance during the rotation of gantry 102 as the above, gantry 102 may be rotated at a constant speed and, at the same time, the CT scanning may be performed to photograph a CT image (910). Once the photographing of the CT image is completed, gantry 102 may be stopped by decreasing the speed thereof (912).

In some embodiments of the invention, the apparatus may be configured to prevent rotation of the body before the fluid bearing gap is created. This prevention may involve, for example, mechanical or electrical interlocks. In some embodiments, the apparatus may be configured to allow rotation of the body after the fluid bearing gap is created. This allowance may involve, for example, mechanical or electrical interlocks. In some embodiments, the apparatus may be configured to stop rotation of the body before the fluid bearing gap is lost. This stopping may involve, for example, mechanical or electrical interlocks.

While example embodiments of the invention have been particularly shown and described, it will be understood by those of ordinary skill in the art that various changes in form and details may be made therein without departing from the scope of the present invention as defined by the following claims.

## Claims

1. A vibration control apparatus, comprising:
a fluid bearing provided around a rotating body to form a fluid bearing gap between the fluid bearing and the rotating body, the said fluid bearing gap comprising a fluid supplied by the fluid bearing; and
a pressure regulator configured to variably control a pressure of the fluid supplied by the fluid bearing, based on imbalance information of the rotating body, to compensate for an imbalance of the rotating body.

2. A vibration control apparatus according to claim 1, wherein,
the fluid bearing is an air bearing; and
the pressure regulator is an electro-pneumatic regulator.

3. A vibration control apparatus according to claim 1 or 2,the fluid bearing provided at least partially around the rotating body to form the fluid bearing gap.

4. A vibration control apparatus according to any preceding claim, wherein the fluid bearing extends completely around the body.

5. A vibration control apparatus according to one of claims 1 to 3, wherein the vibration control apparatus comprises a plurality of fluid bearings, and
wherein a pressure of each of the plurality of fluid bearings is variably controlled in an independent manner.

6. A vibration control apparatus according to claim 5, wherein the fluid bearings are equally spaced around the body.

7. A vibration control apparatus according to claim 5 or 6, wherein the fluid bearings act on the body at equal intervals around the body.

8. A vibration control apparatus according to any preceding claim, wherein the fluid bearing is configured to support a weight of the body.

9. A vibration control method, comprising:
rotating a rotating body of a rotating structure;
forming a fluid bearing gap between the rotating body and a fluid bearing by causing the fluid bearing to supply a fluid to the fluid gap; and
controlling a pressure of the fluid in a variable manner, based on imbalance information of the rotating body, to compensate for imbalance of the rotating body.

10. A vibration control method according to claim 9, wherein the fluid bearing is an air bearing.

11. A vibration control method according to claim 9 or 10, wherein the pressure of the fluid bearing is changed by using a pressure regulator.

12. A vibration control method according to claim 11, wherein the pressure regulator is an electro-pneumatic regulator.

13. A vibration control method according to one of claims 9 to 12, wherein the fluid bearing comprises a plurality of fluid bearings, and
wherein a pressure of each of the plurality of fluid bearings is variably controlled in an independent manner.

14. A computed tomography scanner, comprising:
a rotating body in the form of a gantry configured to generate a computed tomography image while rotating around a test subject, and a vibration control apparatus according to one of claims 1 to 8.
